(19) Europäisches Patentamt
European Patent Office
Office européen des brevets

(11) Publication number: 0 289 203 B1

# (12) EUROPEAN PATENT SPECIFICATION

(45) Date of publication of patent specification: 08.01.92 Bulletin 92/02

(51) Int. Cl.$^5$: C07D 493/04, A61K 31/36

(21) Application number: 88303556.0

(22) Date of filing: 20.04.88

## (54) Antitumor and antiviral compounds of marine origin.

(30) Priority: 29.04.87 US 43700

(43) Date of publication of application: 02.11.88 Bulletin 88/44

(45) Publication of the grant of the patent: 08.01.92 Bulletin 92/02

(84) Designated Contracting States: DE ES FR GB IT

(56) References cited:
TETRAHEDRON LETTERS, vol. 41, no. 16, 1985, Pergamon Press, Oxford, Great Britain; R.J. CAPON and J.K. MACLEOD "Structural and stereochemical studies on marine norterpene cyclic peroxides" pages 3391-3404

(56) References cited:
TETRAHEDRON LETTERS, vol. 26, no. 29, 1985, Pergamon Press, Oxford, Great Britain; Y. KATO, N. FUSETANI, S. MATSUNAGA and K. HASHIMOTO "Bioactive marine metabolites IX. Mycalisines A and B, novel nucleosides which inhibit cell division of fertilized Starfish eggs, from the marin sponge mycale SP" pages 3483-3486

(73) Proprietor: HARBOR BRANCH OCEANOGRAPHIC INSTITUTION, INC.
5600 Old Dixie Highway
Fort Pierce, FL 33450 (US)

(72) Inventor: Munro, Murray H. G., Dr.
121, Watford Street
Christchurch 5 (NZ)
Inventor: Perry, Nigel B., Dr.
4, Cardiff Avenue
Christchurch 2 (NZ)
Inventor: Blunt, John W., Dr.
6, Shirldale Place
Christchurch 2 (NZ)

(74) Representative: Marlow, Nicholas Simon et al
Reddie & Grose 16, Theobalds Road
London WC1X 8PL (GB)

EP 0 289 203 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

This invention relates to new organic compounds which have useful antiviral and antitumor activity. More particularly, this invention relates to new antitumor and antiviral compounds derived from marine organisms, i.e., the marine sponge, of the genus Mycale, family Mycalidae and order Poecilosclerida, their methods of use, and their process for production.

Various tumor related diseases inflict man. Considerable research has been devoted to oncology and antitumor measures. Tumors are common in a variety of mammals and the prevention, control of the growth and regression of tumors in mammals is important to man. The term tumor refers to abnormal masses of new tissue growth which is discordant with the economy of the tissue of origin or the host's body as a whole.

Tumors inflict mammals and man with a variety of disorders and conditions including various forms of cancer and resultant cancerous cachexia. Cancerous cachexia refers to the symptomatic discomfort that accompanies the infliction of a mammal with a tumor. These symptoms include weakened condition of the inflicted mammal as evidenced by, for example, weight loss. The seriousness of cancer is well known, e.g., cancer is second only to heart and vascular diseases as a cause of death in man.

Considerable research and resources have been devoted to oncology and antitumor measures including chemotherapy. While certain methods and chemical compositions have been developed which aid in inhibiting, remitting or controlling the growth of tumors, new methods and antitumor chemical compositions are needed.

Viral diseases also inflict man, plants, insects, and animals. The prevention and control of viral diseases has important health and econonic implications.

Viral diseases contribute to inflictions in humans including common colds, herpes, AIDS (Acquired Immune Deficiency Syndrome) and cancer and the importance of their control is obvious. Also important is the control of viral diseases in animals for economic reasons as well as the ability of such animals to become virus reservoirs or carriers which facilitate the spreading of viral diseases to humans. Viral plant diseases have been known to have a disruptive effect on the cultivation of fruit trees, tobacco, and various vegetables. Insect viral diseases are also of interest because of the insects' ability to transfer viral diseases to humans.

The prevention and control of viral diseases is thus of prime importance to man and considerable research has devoted to antiviral measures. Certain methods and chemical compositions have been developed which aid in inhibiting, controlling or destroying viruses but additional methods and antiviral chemical compositions are needed.

Marine organisms and particularly marine sponges are a potential source for chemically and biologically interesting molecules of great diversity. Some such molecules derived from sponges are described in Scheuer, P.J. Ed., Marine Natural Products, Chemical and Biological Perspectives ; Academic Press ; New york, 1978-1983 ; Vol. I-V ; Kato et al., Tetrahedron Letters, Vol. 26, Pg. 3483-6 (1985) ; and Capon and Macleod, Tetrahedron, Vol. 41, Pg. 3391-3404 (1985).

Another naturally derived compound of interest is pederin. Pederin is isolated from insects of the Paederus genus. Pederin shows antimitotic activity as described in British Patent Specification Nos. 1,078,049 (1967) and 932,875 (1963).

It has now been found that certain compounds derived from extracts of marine sponge of the genus Mycale, family Mycalidae, and order Poecilosclerida, possess useful antitumor and antiviral activity.

The invention provides novel compounds which are useful as antiviral and antitumor agents and a process for producing such novel antitumor and antiviral compounds.

According to the present invention there is provided a compound of the general formula (I) :

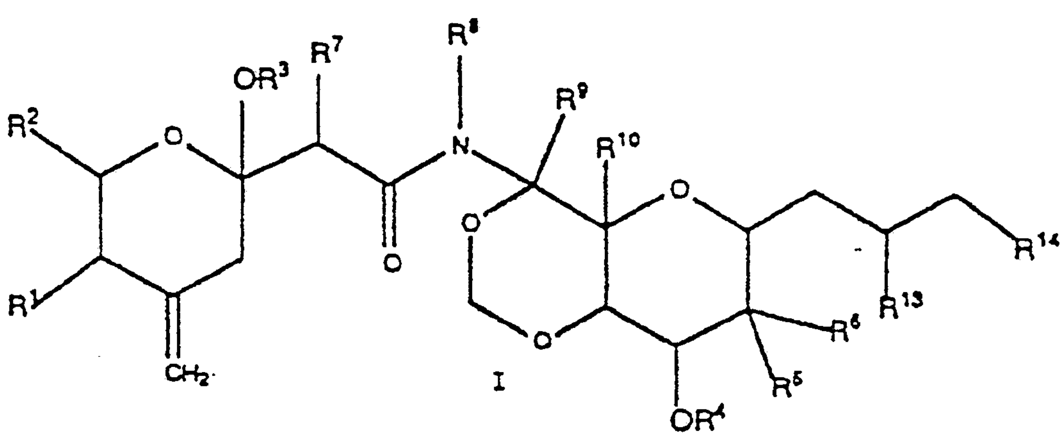

I

wherein $R^{1-6}$ are the same or different and are a hydrogen, or methyl ;
and $R^{7-14}$ are the same or different and are hydrogen or hydroxy groups.

In preferred embodiments of the invention, the compound is substantially pure.

In preferred embodiments of the invention $R^{1-6}$ are methyl groups.

In more preferred embodiments of the invention, the invention comprises a compound named mycalamide and has the formula (II) :

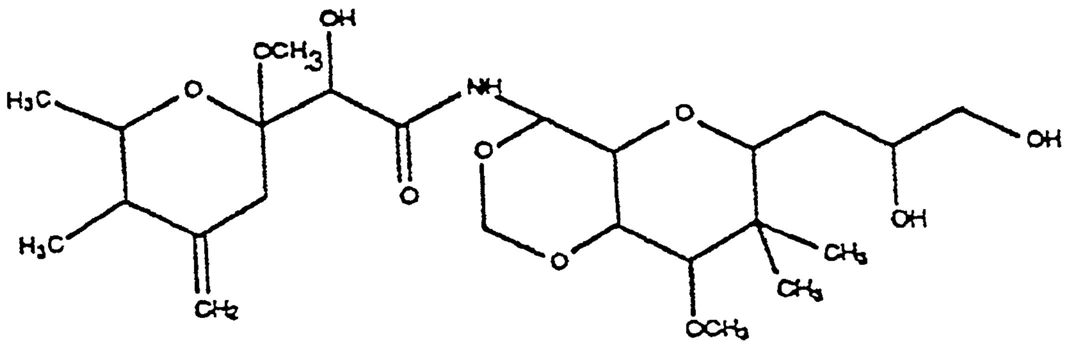

II

As embodied and fully described herein, the invention also comprises an antiviral and antitumor composition comprising, as active ingredient, an effective antitumor or antiviral amount of one or more compound according to formulae I or II and a non-toxic pharmaceutically acceptable carrier or diluent.

As embodied and fully described herein, the invention also comprises a process to produce the compounds of formulae I and II. The process comprises the steps of : collecting marine sponge of the genus Mycale, family Mycalidae, and order Poecilosclerida ; contacting the sponge with at least one suitable organic solvent to obtain an extract comprising a compound according to formula I or II ; and isolating a compound according to formula I or II from the extract.

In preferred embodiments of the invention the suitable organic solvent is selected from the group consisting of methanol, toluene, ethyl acetate, hexanes, heptane, isooctane, isopropanol, ethanol, benzene, acetone, diethyl ether, t-butyl-methyl ether, chloroform, 1,2-dichloroethane, dicholoromethane, and combinations thereof.

Reference will now be made in detail to present preferred embodiments of the invention, examples of which are illustrated in the following example section.

In accordance with the invention, an antitumor composition is provided comprising as active ingredient an effective antitumor amount of one or more of the compounds described above and identified by formulae I or II and a non-toxic pharmaceutically acceptable carrier or diluent. While effective amounts may vary, as conditions in which the antitumor compositions are used vary, a minimal dosage required for activity is generally between 0.001 and 1.0 micrograms against $10^5$ tumor cells. Useful examples of non-toxic pharmaceutically acceptable carriers or diluents include, but are not limited to, the following : ethanol, dimethyl sulfoxide and glycerol.

A method for inhibiting tumors in a host is provided comprising contacting a tumor with an effective antitumor amount of one or more compounds according to formulae I or II. The effectiveness of the compounds of the invention for inhibiting tumors and tumor cells indicates their usefulness for controlling tumors in hosts, including mammals, and for treating cancerous cachexia.

In accordance with the invention, an antiviral composition is provided comprising as active ingredient an effective antiviral amount of one or more of the compounds described above and identified by formulae I or II and a non-toxic pharmaceutically acceptable carrier or diluent. While effective amounts may vary, as conditions in which the antiviral compositions are used vary, a minimal dosage required for activity is generally between 0.002 and 2.0 micrograms against 25-80 plaque-forming units of virus. Useful examples of non-toxic pharmaceutically acceptable carriers or diluents include, but are not limited to, the following : ethanol ; dimethyl sulfoxide ; and glycerol.

Virus cells are inhibited or killed by a method comprising contacting a virus with an effective antiviral amount of one or more compounds according to formulae I or II. The minimal effective amount as stated above is generally from 0.002 to 2.0 micrograms against 25 to 80 plaque forming units of virus. The compounds of formulae I or II are active for inhibiting or killing a diverse range of viruses including, but not limited to, the RNA viruses, Vesicular stomatitis (herein "VSV"), arenaviruses, coronaviruses, rhinoviruses, influenza viruses and the DNA

viruses, Herpes simplex-I (herein "HSV-I"), other Herpes viruses, adenoviruses, coxsackie viruses, polioviruses and papovaviruses.

The effectiveness of the compounds of the invention for inhibiting virus cells indicates that the compounds of formulae I or II should also be useful in controlling viral infections in host animals and plants which are caused by a virus which is thus inhibited or destroyed. Viral infections which may be controlled by utilizing compositions of the present invention include, but are not limited to, those caused by those RNA viruses and DNA viruses described above. The invention may also be useful in controlling common viral infections of plants.

A detailed description and explanation of a preferred embodiment of the process of the invention to produce the compounds according to formulae I or II is as follows : the marine sponge genus Mycale, family Mycalidae, and order Poecilosclerida is collected from the channel at Aquarium Point, Otago Harbour, New Zealand. The marine sponge is extracted by blending with a 3 : 1 mixture of methanol : toluene to obtain a liquid extract which is concentrated to yield a crude residue. The residue is then fractionated by reverse phase flash chromatography with a suitable solvent system, for example, a gradient from water to methanol.

While a mixture of methanol and toluene is the presently preferred choice for the extracting solvent, other suitable solvents may be substituted. A suitable solvent should be capable of extracting a compound according to any one of formula I or II from other components of the marine sponge. Suitable solvents which may be substituted for methanol or toluene include but are not limited to hexanes, heptane, isooctane, ethyl acetate, isopropanol, ethanol, benzene, acetone, diethyl ether, t-butyl-methyl ether, chloroform, 1,2-dichloroethane, dicholoromehthane, and combinations thereof.

Any suitable fractionation and isolation technique may be utilized in accordance with the process of the invention. Suitable fractionation techniques include reverse phase chromatography utilizing a suitable separator-extractor column as described in Blunt et al., J. Hat. Prod., Vol. 50, in press (1987), the entire disclosure of this reference is hereby incorporated herein by reference. These columns are eluted with suitable eluents such as : water ; methanol ; heptane ; hexanes ; ethyl acetate ; chloroform ; acetonitrile ; and n-propanol ; n-butanol and various combinations and ratios thereof as would be known to those skilled in the art.

## EXAMPLES

The invention will now be illustrated by example. The example is not intended to be limiting of the scope of the present invention. In conjunction with the detailed and general description above, the example provides further understanding of the present invention and outlines a process for producing compounds of the invention.

The following example represents a preferred embodiment of the compounds processes and methods of the invention.

The starting materials and reagents in the example whose method of preparation are not indicated are commercially available from sources known to the art such as chemical supply houses.

### Example 1

The antitumor and antiviral mycalamide of the invention has prepared according to the following procedures.

Preparation of Mycalamide :

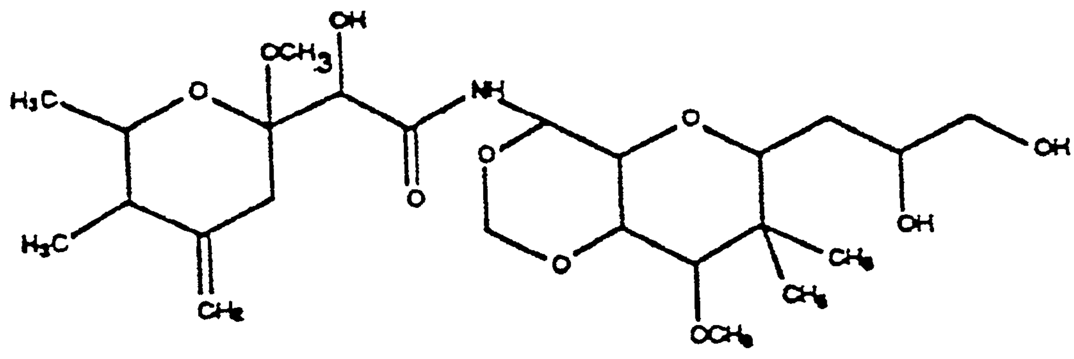

Compound 1

A new Zealand marine sponge of the genus Mycale (family Mycalidae, order Poecilosclerida), was collected from the channel at Aquarium Point, Otago Harbour New Zealand. 200 gms. of frozen sponge was extracted by blending with 3 : 1 methanol : toluene and filtering off the solid. On removing the solvents the combined extracts from three such steps yielded a brown gum (11.04 gms) with antiviral properties. Reverse phase flash chromatography (Blunt et al., J. Nat. Prod., 50 (1987) in press) gave bioactive fractions or eluting with 1 : 1, 3: 1 and 9 : 1 of $H_2O$ : methanol. These were combined to give a brown oil (307 mg). A subsample of this material (140 mg) was applied to a column of Fractogel (Trademark) PGM 2000 (120 gms Fractogel, column 43 cm × 2 cm). Eluting with 1 : 4, $H_2O$ : methanol (0,5 ml/min) gave bioactive fractions at around 1.5 void volumes, which were combined (brown oil, 50 mg). Silica gel column chromatography (DAVISIL (Trademark), 35-60 μ, 5 gms) starting with $CH_2Cl_2$, then increasing amounts of methanol, gave 1.7 mg of mycalamide (1) in the most bioactive fraction, eluted with 1 : 9 methanol ; $CH_2Cl_2$.

Spectral data for mycalamide (1) :

[α] 365 nm + 110° (c 0.2 gm/100 ml, $CHCl_3$)

| | |
|---|---|
| MS (EI) : | M+, measured 503.27220 daltons, calculated for $C_{24}H_{41}NO_{10}$ 503.27305 (– 1.7 ppm). M+-Methanol, measured 471.24824 daltons, calculated for $C_{23}H_{37}NO_9$ 471.24683 (+ 3.0 ppm). |
| IR, film, (cm-1) : | 3700-3100, 2690, 1740, 1540, 1470, 1390, 1100, 1080, 1040. |

$^1H$ nmr:

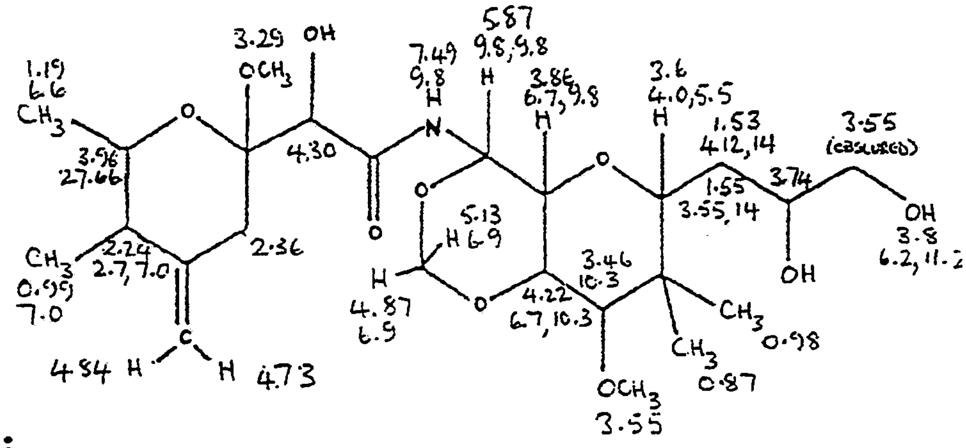

$^{13}C$ nmr:

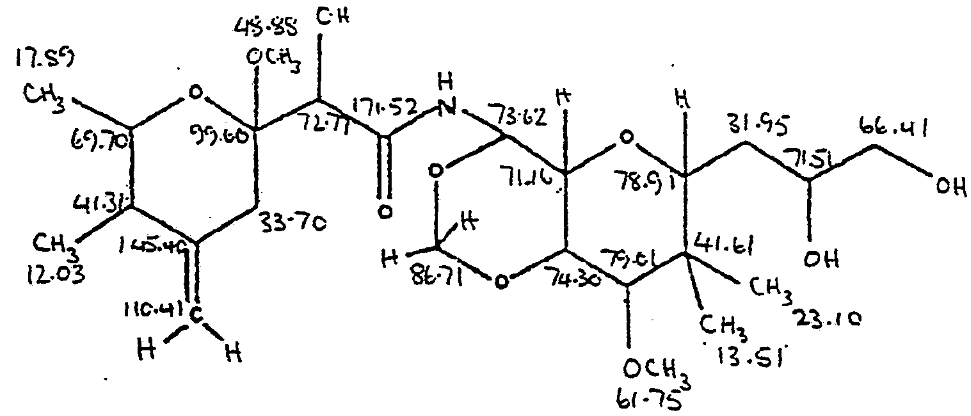

ANTITUMOR ACTIVITIES OF THE COMPOUNDS OF THE INVENTION

The following assay method was utilized to illustrate the antitumor effectiveness of the compounds of formulae I and II, corresponding to compound 1 (mycalamide) of the example.

P388 MOUSE LEUKEMIA CELL ASSAY

Maintenance of Cell Line

P388 mouse leukemia cells are grown in Dulbecco (Trademark) MEM medium with 10% horse serum, 4 mM glutamine, and 20 ug/ml gentamycin (Biologos, Inc.). Cells are incubated in 10% $CO_2$ and subcultured 2 times per week.

In vitro PROCEDURE

1. Add compound to each well of a 24-well plate or tube and allow solvent to evaporate to dryness.
2. Add 2 ml ($1.2 \times 10^5$) cells to each well or tube and mix.
3. Incubate in 10% $CO_2$ at 35° for 48 hours.
4. Read plates with an inverted microscope, scoring activity from 1+ to 4+ as follows : ND (not detectable),>90% ; 1+, 75-90% ; 2+, 50-74% ; 3+, 25-49% ; 4+, < 25% of control growth. Cell counts are performed on each tube and results are reported as percent of control.

P338 In vivo Procedure

1. Compounds are dissolved or suspended in sterile physiological saline with a drop of Tween (Trademark) detergent added to suspensions, if necessary. Standard lab mice are injected IP with a tuberculin syringe with a 23-gauge needle. The injection volume is 0.1 ml.

2. Test compound is injected starting 24 hours after P338 tumor implantation. The treatment is repeated daily for eight days, less injection if limited by quantity.
3. Animals are closely observed for the first hour after each injection. Animals are checked daily and weighed on Day 5 and Day 30.
4. Testing with positive control compound is scheduled for every other experiment. The dose is 20 mg/kg for 9 days. The lowest acceptable T/C value is 135%. The acceptable control median survival time is 9-13 days.
5. The T/C value is calculated using the formula

$$\% \ T/C = \frac{\text{Median survival of test group}}{\text{Median survival of control group}} \times 100\%$$

T/C value of > 130% is considered significant.

Positive control — Vinblastine or Vincristine in aqueous solution.

Procedure for B16 Murine Melanoma Assay

B16 melanoma is maintained in vivo by serial subcutaneous passage in mice. For this assay, tumor obtained from mice was inoculated ip into test mice. The Inoculum level was 0.5 ml of a 10% tumor brei. Twenty-four hours post-Inoculation mice were randomized into groups of ten. Bacteriological check of tumor was negative. Test materials were dissolved or suspended in sterile 0.9% NaCl solution (plus minimal amounts of ethanol and Tween-80 as needed) and administered ip daily in a volume of 0.5 ml/mouse. Treatment has begun 24 hours post-implant and continued for 5 to 9 days depending on quantity of material available. Mice were weighed on days 1 and 5 and deaths recorded daily. End points for therapeutic evaluations were mean and median survival time and long-term survivors on day 60.

HUMAN TUMOR CELL LINE ASSAY

Maintenance of Cell Line

HCT-8 human colon tumor cells are grown in RPMI 1640 medium (GIBCO). A-549 human lung carcinoma cells are cultured in Dulbecco medium (Biologos, Inc.). MCF-7, MDA-MB 231 and T47D human breast carcinoma cells are grown in Eagles MEM medium. All media are supplemented with 10% fetal bovine serum and contain 50 ug/ml gentamycin. All human tumor cell lines are incubated in 5% $CO_2$ at 37° and subcultured once a week. The seeding levels are 350,000 MCF-7 cells, 60,000 HCT-8 cells and 300,000 A-549 cells per T25 flask.

PROCEDURE

1. Seed 1 ml of cells (5000 HCT-8, 8000 A549, 12000 MCF-7) into each well of a 24-well plate.
2. Incubate in a $CO_2$-incubator for 48 hours.
3. Add compound (50 µg) to each well and incubate for an additional 120 hours.
4. Discard medium and stain with methylene blue (HCT-8) or crystal violet (A549 and MCF-7) for 30 minutes.
5. Compare cell density of drug-treated well with that of the control (no drug added) as follows : ND (not detectable), > 90% ; 1+, 75-90% ; 2+, 50-74% ; 3+, 25-49%, 4+, < 25% of control growth.

Final Conc. of Vinblastine control
(use 2 ul/assay)

| Solution Conc. | Amt added | Final conc. in test |
|---|---|---|
| 50 mg/ml | 2 µl | 50 µg/ml |
| 20 mg/ml | 2 µl | 20 µg/ml |
| 5.0 mg/ml | 2 µl | 5.0 µg/ml |
| 0.5 mg/ml | 2 µl | 0.5 µg/ml |

ANTIVIRAL ACTIVITIES OF THE COMPOUNDS OF THE INVENTION

The following assay method was utilized to demonstrate the in vitro antiviral effectiveness of the compound of formulae I and II, corresponding to compound 1 of the example.

Antiviral Disc Assay for HSV-1 and VSV

A. Maintenance of Cell Cultures

1. Virus
a. Both Herpes simplex type 1 (HSV-1) and Vesicular stomatitis virus (VSV) replicates in the CV-1 cell line. CV-1 is a fibroblast-like cell culture derived from primary African green monkey cells.
2. Growth of CV-1 Cells
   a. Seed 150 $cm^2$ tissue culture flasks each with $10 \times 10^6$ CV-1 cells in 40 ml of EMEM with 10% FBS (growth medium).
   b. Seven days after seeding the flasks cell numbers should be approximately $40\text{-}50 \times 10^6$ cells. CV-1 cells have a doubling time of 72 hours based on these numbers.
3. Trypsinization
   a. Aseptically remove the medium
   b. Rinse cell sheet two times with 10 ml of $Ca^{++}$ and $Mg^{++}$ free Dulbecco's phosphate buffered saline.
   c. Add 4.0 ml of trypsin — EDTA mixture.
   d. Incubate flask at room temperature with occasional rocking for 5 minutes.
   e. Shake flash.
   f. Add 10 ml EMEM growth medium and break up cell clumps with pipetting.
   g. Count cells.

B. Preparation of plates for viral assays

1. Cell Concentration
a. Dilute the cells with EMEM to $4 \times 10^5$ cells/ml.
b. Seed 24 well trays with 0.5 ml per well. Cell concentration per well is $2 \times 10^5$ cells.
c. Incubate at 37°C with 5% $CO_2$.
d. The wells can be used over the next several days beginning the day after seeding (preferably 2, 3, or 4).

C. Assay of HSV-1 and VSV in CV-1 cells

1. Infection of CV-1 cells in plates with virus
   a. Remove medium from wells.
   b. Infect well with at least 25 and no more than 80 plaque forming units (PFU) of virus.
   c. Incubate infected cells at 37°C for 1.0 hour.
   d. Pour off supernatant at end of incubation period.
   e. Add 0.5 ml of methylcellulose overlay medium (MCO).
1) MCO is a maintenance medium without phenol red made with 1% 4000 centipose methylcellulose. FBS is used at 5% level.

2. Drug Evaluation

a. For drug evaluation wet filter paper discs (6 mm diameter) with approximately 0.02 ml of marine extract or test compound.

1) Allow solvent to evaporate for 20 to 30 minutes at room temperature.

2) Place discs in the well containing CV-1 cells, virus, and MCO.

b. Incubate tissue culture plates for 48 hours at 37° C.

c. After 48 hours place 0.5 ml NRMCO on each well.

1) NRMCO is a maintenance overlay medium without phenol red containing 0.1 mg neutral red dye per ml and 2% 15 centipose methylcellulose.

d. Incubate plates at 37°C and read the following day.

1) Antiviral activity should be observed from two parameters. One is actual reduction in the number of plaques and two is the diminution in plaque diameter.

3. Scoring Drug Activity

a. Antiviral activity (AVA) is scored from 0 to +++.

| | |
|---|---|
| +++ = | complete inhibition of plaque formation |
| ++ = | partial inhibition |
| + = | partial inhibition |
| 0 = | no protection |

b. Cytotoxicity

Wells of 24 well tissue culture plates are 16 mm in diameter. Discs are 6 mm in diameter. Zones of cytotoxicity greater than 6 mm are graded from 8 to 16 using only even numbers.

| | |
|---|---|
| 0 = | no macroscopic or microscope cytotoxicity |
| 16 = | 100% toxicity or complete cell destruction |
| 8, 10, 12, 14 = | partial cytotoxicity, i.e. diameter of toxic zone including diameter of 6 mm disc. |

A59 coronavirus tests were conducted in vitro as above, and in vivo in mice as described below.

A59 In Vivo Assay Procedure

Four-week-old mice are inoculated intraperitoneally with 42 $LD_{50}$ doses of virus. Compound is administered on same day and for 9 consecutive days thereafter. Mice are held for observation for fourteen days total. Mice inoculated with virus only are expected to die in 3 to 7 days with a mortality rate of 80 to 100%. A reading of 0% mortality in mice receiving drug plus virus indicates protection from gross clinical disease and death.

The results of the above described in vivo and in vitro assays are summarized in Tables 1 and 2.

Antitumor and Antiviral Activity of Mycalamide (1)

Table 1: Antiviral Activity

Table 1A in vitro activity

| Dose (ug/well)* | VSV | | HSV-1 | | A-59 | |
|---|---|---|---|---|---|---|
| | Cyt | AVA | Cyt | AVA | Cyt | AVA |
| 40 | 16 | | 16 | | 8 | +++ |
| 20 | 16 | | 16 | | 8 | +++ |
| 2 | 16 | | 16 | | 8 | +++ |
| 0.2 | 12 | ++ | 12 | ++ | 0 | ++/+++ |

Table 1B in vivo activity against A-59 coronavirus.

| Mice | | Drug conc.* (mg/kg) | Cumulative Mortality Group Days after Infection 2 | 4 | 6 | 8 | 10 | 12 | 14 |
|---|---|---|---|---|---|---|---|---|---|
| Drug | 4 | 1 | 0 | 0 | 100 | 100 | 100 | 100 | 100 |
| Drug+Virus | 4 | 1 | 0 | 0 | 75 | 100 | 100 | 100 | 100 |
| Drug | 4 | 0.1 | 0 | 0 | 0 | 0 | 0 | 0 | 0 |
| Drug+Virus | 4 | 0.1 | 0 | 0 | 0 | 0 | 0 | 0 | 0 |
| Drug | 4 | 0.01 | 0 | 0 | 0 | 0 | 0 | 0 | 0 |
| Drug+Virus | 4 | 0.01 | 0 | 0 | 25 | 25 | 25 | 25 | 25 |
| Virus only | 8 | | 0 | 63 | 88 | 100 | 100 | 100 | 100 |
| Ribavirin (control) +Virus | 4 | 100 | 0 | 0 | 0 | 0 | 0 | 0 | 0 |

• Table 2: Antitumor Activity

Table 2a in vitro Mouse P 388 assay

$IC_{50}$ = 0.0016 ug/ml

Table 2b in vivo P 388 in mice

| Dose mg/kg | Median T/C % | |
|---|---|---|
| | 1st series | 2nd series |
| 0.02 | Toxic | 106 |
| 0.01 | 116 | 156 |
| 0.005 | 132 | 133 |
| 0.0025 | 137 | 122 |
| 0.0012 | | 117 |

Table 2c in vivo B-16 melanoma assay in mice

| Dose mg/kg* | Median T/C % | Cures |
|---|---|---|
| 0.5 | Toxic | 0 |
| 0.25 | 214 | 1 |
| 0.125 | 189 | 0 |
| 0.0625 | 197 | 0 |

* These assays were carried out on crude (2%) mycalamide extracts and such is reflected in the dose levels.

Table 1 shows that mycalamide has antiviral activity in vitro and in vivo. It is apparent from this in vitro and in vivo testing that the compounds of the invention are effective for inhibiting viral growth and therefore for controlling viral related diseases in hosts including mammals and plants such as herpes and the common cold.

It is apparent from the in vitro and in vivo testing and results reported in Table 2 that the compounds of the invention are effective for inhibiting or destroying tumors and therefore in controlling diseases caused by or related to such tumors in hosts, including mammals, such as cancerous cachexia.

**Claims**

1. A compound according to the general formula :

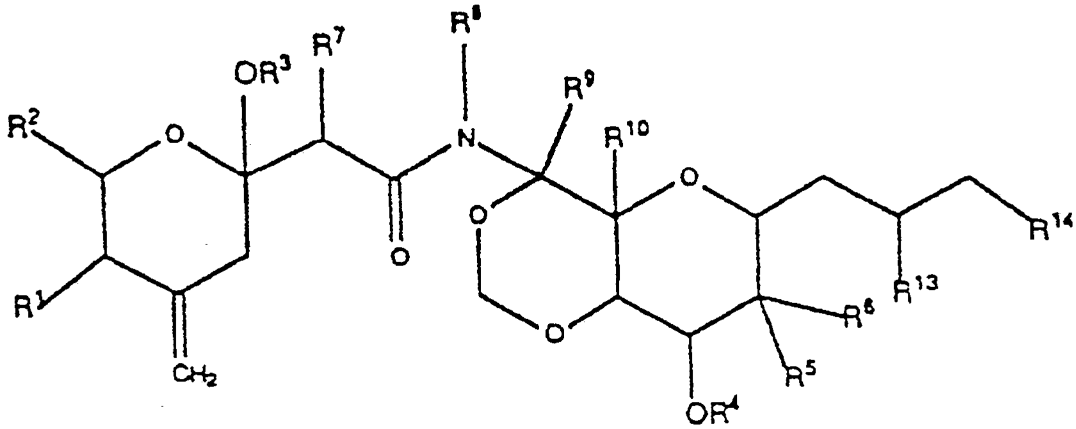

wherein $R^{1-6}$ are the same or different and are a hydrogen or methyl ; and $R^{7-14}$ are the same or different and are single bonded hydrogen or hydroxy groups.

2. A compound according to claim 1 wherein $R^{1-6}$ are methyl groups.

3. A compound according to claim 1 or 2 of the formula :

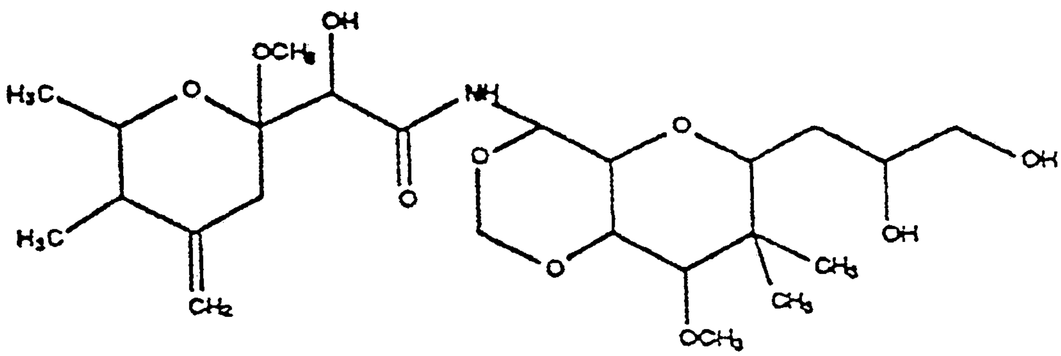

4. A compound according to claim 1, 2 or 3 wherein said compound is substantially pure.

5. An antitumor composition comprising, as active ingredient, an effective antitumor amount of one or more of the compounds of any of claims 1 to 4 and a non-toxic pharmaceutically acceptable carrier or diluent.

6. An antiviral composition comprising, as active ingredient, an effective antiviral amount of one or more of the compounds of claims 1 to 4 a non-toxic pharmaceutically acceptable carrier or diluent.

7. A Process to produce a compound according to any one of claims 1 to 4 comprising the steps of :
collecting marine sponge of the genus Mycale, family Mycalidae and order Poecilosclerida ;
contacting said sponge with suitable organic solvents ;
obtaining an extract of the sponge and solvent mixture ; and
isolating a compound according to claim 1 from the extract.

8. A process according to claim 7 to produce a compound according to claim 3 comprising the steps of :
collecting marine sponge genus Mycale, family Mycalidae, and order Poecilosclerida ; contacting said sponge with a suitable organic solvent selected from the group consisting of methanol, toluene, hexanes, heptane, isooctane, isopropanol, ethanol, benzene, ethyl acetate, acetone, diethyl, ether, t-butyl-methyl ether, chloroform, 1, 2-dichloroethane, dicholoromeththane, and mixtures thereof ;
obtaining an extract of the sponge and solvent mixture ; and
isolating a compound according to claim 3 by reverse phase flash chromatography from the extract.

**Patentansprüche**

1. Verbindung der allgemeinen Formel

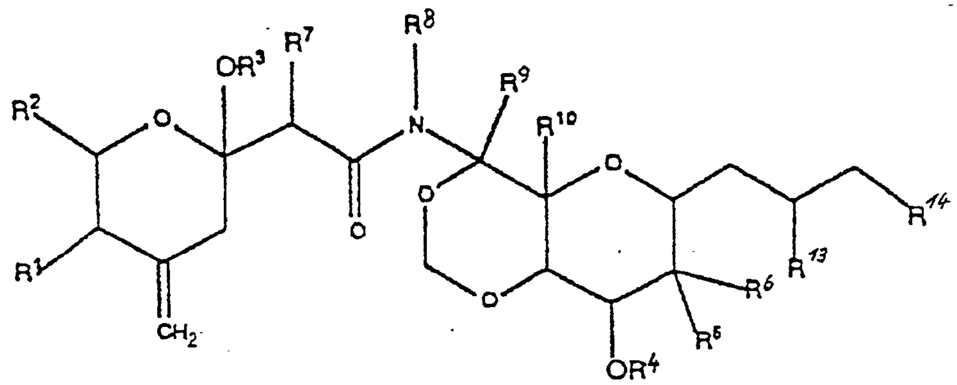

worin $R^{1-6}$ gleich oder verschieden sein können und ein Wasserstoffatom oder eine Methylgruppe bedeuten und $R^{7-14}$ gleich oder verschieden sein können und einfach gebundene Wasserstoffatome oder Hydroxylgruppen bedeuten.

2. Verbindung gemäß Anspruch 1, worin $R^{1-6}$ Methylgruppen bedeuten.

3. Verbindung gemäß Anspruch 1 oder 2 der Formel

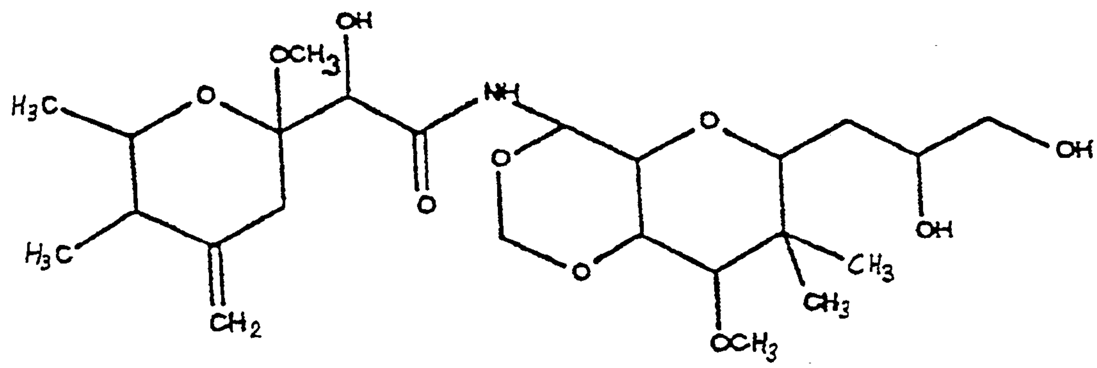

4. Verbindung gemäß Anspruch 1, 2 oder 3, worin die Verbindung praktisch rein ist.

5. Antitumormittel, enthaltend eine zur Tumorbekämpfung wirksame Menge einer oder mehrerer der Verbindungen gemäß einem der Ansprüche 1 bis 4 als aktiven Bestandteil sowie ein ungiftiges, pharmazeutisch annehmbares Träger- oder Verdünnungsmittel.

6. Antivirales Mittel, enthaltend eine zur Virus-bekämpfung wirksame Menge eines oder mehrerer der Verbindungen gemäß Anspruch 1 bis 4 als aktiven Bestandteil sowie ein ungiftiges, pharmazeutisch annehmbares Träger- oder Verdünnungsmittel.

7. Verfahren zur Herstellung einer Verbindung gemäß einem der Ansprüche 1 bis 4, bei dem man
einen Meeresschwamm des Genus Mycale, der Familie Mycalide und der Ordnung Poecilosclerida sammelt;
den Schwamm mit geeigneten organischen Lösungsmitteln in Berührung bringt;
einen Extrakt des Schwammes und ein Lösungsmittelgemisch erhält und
aus dem Extrakt eine Verbindung gemäß Anspruch 1 isoliert.

8. Verfahren gemäß Anspruch 7 zur Herstellung einer Verbindung gemäß Anspruch 3, wobei man
einen Meeresschwamm des Genus Mycale, der Familie Mycalide und der Ordnung Poecilosclerida sammelt;
den Schwamm mit einem geeigneten organischen Lösungsmittel aus der Gruppe Methanol, Toluol, Hexan, Heptan, Isooctan, Isopropanol, Ethanol, Benzol, Ethylacetat, Aceton, Diethylether, t-Butyl-methyl- ether, Chloroform, 1,2-Dichlorethan, Dichlormethan oder einem Gemisch daraus in Berührung bringt;
einen Extrakt des Schwammes und ein Lösungsmittelgemisch erhält und
aus dem Extrakt eine Verbindung gemäß Anspruch 3 durch Phasenumkehr-Blitzchromatographie isoliert.

**Revendications**

1. Composé répondant à la formule générale :

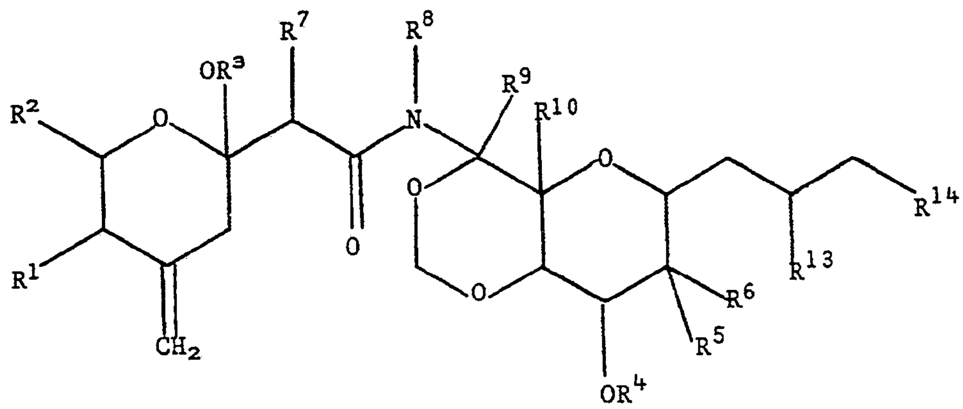

dans laquelle les symboles $R^1$ à $R^6$ sont semblables ou différents et sont un atome d'hydrogène ou un groupe méthyle et les symboles $R^7$ à $R^{14}$ sont semblables ou différents et sont des atomes d'hydrogène ou des groupes hydroxy à simple liaison.

2. Composé selon la revendication 1, dans lequel les symboles $R^1$ à $R^6$ sont des groupes méthyles.

3. Composé selon la revendication 1 ou 2 de formule :

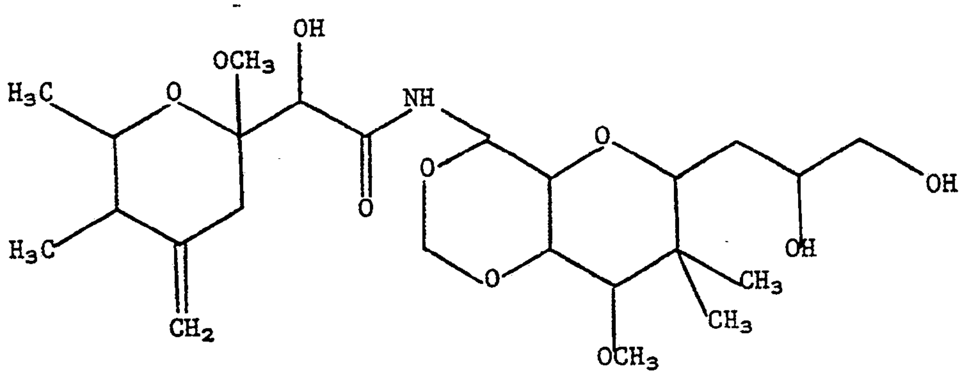

4. Composé selon la revendication 1, 2 ou 3, ledit composé étant sensiblement pur.

5. Composition antitumorale comprenant comme ingrédient actif une quantité antitumorale efficace d'un ou plusieurs des composés de l'une quelconque des revendications 1 à 4 et un véhicule ou diluant non toxiques pharmaceutiquement acceptables.

6. Composition antivirale comprenant comme ingrédient actif une quantité antivirale efficace d'un ou plusieurs des composés des revendications 1 à 4 et un véhicule ou diluant non toxiques phamaceutiquement acceptables.

7. Procédé pour préparer un composé selon l'une quelconque des revendications 1 à 4 comprenant les étapes de :

récolte d'une éponge marine du genre Mycale, famille Mycalidae et ordre Poecilosclerida ;

contact de ladite éponge avec des solvants organiques appropriés ;

obtention d'un mélange d'extrait de l'éponge et de solvant ; et

isolement d'un composé selon la revendication 1 à partir de l'extrait.

8. Procédé selon la revendication 7 pour préparer un composé selon la revendication 3 comprenant les étapes de :

récolte d'une éponge marine du genre Mycale, famille Mycalidae et ordre Poecilosclerida ;

contact de ladite éponge avec un solvant organique approprié choisi dans le groupe constitué par le méthanol, le toluène, les hexanes, l'heptane, l'isooctane, l'isopropanol, l'éthanol, le benzène, l'acétate d'éthyle, l'acétone, l'éther diéthylique, l'éther tert-butylméthylique, le chloroforme, le 1,2-dichloroéthane, le dichlorométhane et leurs mélanges ;

obtention d'un mélange d'extrait de l'éponge et de solvant ; et
isolement d'un composé selon la revendication 3 par chromatographie éclair en phase inverse à partir de l'extrait.